# EUROPEAN PATENT APPLICATION

(11) **EP 2 468 705 A1**
(43) Veröffentlichungstag der Anmeldung: **27.06.2012**
(21) Anmeldenummer: 09848538.6
(22) Anmeldetag: 16.11.2009
(51) Int. Cl.: C07C 27/00, C07C 31/00, C07C 47/04

(54) **PFLANZE ZUR HOMOGENEN OXIDATION VON METHANHALTIGEM GAS UND VERFAHREN ZUR OXIDATION VON METHANHALTIGEM GAS**

(30) Priorität: 19.08.2009 RU 2009131339
(71) Anmelder: Otkrytoe Akcionernoe Obwestvo "GTL", Moscow 117420 (RU)
(72) Erfinder: BLINICHEV, Valer'jan Nikolaevich, Ivanovo 153002 (RU); KADYROV, Rafis Faizovich, Respublika Bashkortostan (RU); CHAGIN, Oleg Vjacheslavovich, Ivanovo 153022 (RU)
(74) Vertreter: Jeck, Anton
(86) Internationale Anmeldenummer: PCT/RU2009/000625
(87) Internationale Veröffentlichungsnummer: WO 2011/021955

(57) **Zusammenfassung**

Die Erfindung betrifft ein Verfahren zur homogenen Oxidation von methanhaltigem Gas. Das Verfahren besteht aus Folgendem: Das methanhaltige Gas wird bis auf 430 - 450° C vorgewärmt und in wenigstens drei in Reihe angeordnete Reaktoren (7, 8, 9) für eine Oxidation gefördert. Dabei wird jeder der Reaktoren (7, 8), bis auf den letzten (9), jeweils mit Abhitzkesseln (14, 15) verbunden. Dem Reaktor (7) wird auch Sauerstoff für die Oxidation des methanhaltigen Gases zugeführt. Dabei wird die Temperatur der Gasmischung auf 540 - 560° C erhöht. Nachfolgend erfolgt eine schnelle Abschreckung/Kühlung der Gasmischung in den Abhitzkesseln (7, 8) bis auf 440 - 450° C. Dabei wird Dampf gebildet, welcher in die Rektifikationssäule (12) zur Trennung der Endprodukte gefördert wird. Das Reaktionsgemisch aus dem letzten Reaktor (9) fließt in einen Abscheider (10). Von hier aus fließt die flüssige Phase zur Rektifikationsstufe (12), wo Methanol-Rektifikat, Äthanol, Formaldehyd erzeugt werden. Die Gasphase fließt dagegen zur Gasreinigung und Entfernung von SO₂, CO, CO₂. Dabei wird ein teilweises Abblasen des Umlaufzyklus durchgeführt, um die inerten Gase zu entfernen. Nach der Reinigung und dem Abblasen erfolgt der Rückschluss des Zyklus mittels der Zuspeisung der Gasphase/des Umlaufgases in das methanhaltige Grundgas. Das neu gebildete Gas wird wieder dem Reaktor (7) zugeführt. Die Erfindung ermöglicht es, den Nutzeffekt zu steigern, die Ausbeute des Endprodukts zu erhöhen und die Umweltbedingungen zu verbessern. Zudem betrifft die Erfindung eine Anlage zur homogenen Oxidation von methanhaltigem Gas zur Durchführung des Verfahrens.

## Beschreibung

Die Erfindung betrifft ein Verfahren zur homogenen Oxidation von methanhaltigem Gas nach dem Oberbegriff des Anspruchs 1 und eine Anlage zur Durchführung des Verfahrens.

Diese Erfindung ist in der petrolchemischen Industrie, insbesondere bei einem Verfahren zur homogenen Oxidation von methanhaltigem Gas sowie bei einer Anlage zur homogenen Oxidation von methanhaltigem Gas einsetzbar.

Die Erdgasreserven sind groß. Erdgas ist ein umweltfreundlicher Brennstoff. Jedoch ist die direkte Nutzung des gasförmigen Energieträgers oft und insbesondere bei Verkehrsmitteln ungünstig. Das Problem wird dadurch gelöst, dass das Erdgas in einen bequemeren flüssigen Brennstoff umgewandelt wird. Das kann insbesondere Methanol sein, welches dazu noch als Grundstoff für viele chemische Produkte verwendet wird.

Aus dem Stand der Technik sind mehrere Verfahren zur Verwandlung von Methan zu Methanol bekannt. Das Dampfreformieren von Methan (SMR Technology) zu Synthesegas (Gemisch von CO und H₂) mit seiner nachfolgenden Kontaktumwandlung zu Methanol ist weit verbreitet und verwendet (M.M. Karavaev, V.E. Leonov und a. m. "Synthetisches Methanolverfahren". - M.: "Chimiya", 1984, S. 72 - 125). Jedoch weist dieser Vorgang einige wesentliche Mängel auf: komplizierte Ausrüstung, hohe Anforderungen an die Gasreinheit, großer Energieaufwand für die Produktion von Synthesegas und seine Reinigung, zahlreiche Zwischenstufen beim Produktionsvorgang und Unwirtschaftlichkeit von Klein- und Mittelproduktionen mit einer Produktionsleistung von unter 2000 t/Tag.

Zurzeit ist die direkte Gasphasenoxidation von Methan zu Methanol unter hohem Drücken von höchstem Interesse. Dabei wird auf die Produktionsstufe der Herstellung von Synthesegas verzichtet. Der Vorgang verläuft unter Drücken bis zu 10 MPa und Temperaturen von 400 - 450° C in Rohrreaktoren bei relativ geringen Sauerstoffkonzentrationen. Anschließend wird das Gas-Flüssigkeitsgemisch gekühlt, und die flüssigen Produkte werden ausgeschieden. Dabei wird Methanol mittels Rektifikation ausgeschieden (V.S. Arutyunov, O.V. Krylov "Oxidative Umwandlungen von Methan" - M.: "Nauka", 1998, S. 130 - 145). Jedoch hemmen der niedrige Grad der Methanreformierung pro Passage durch einen Reaktor von max. 3 **-** 5 % und dementsprechend die niedrige Ausbeute von Methanol den praktischen Einsatz des Verfahrens zur Herstellung von Methanol durch eine direkte Oxidation von Methan.

Unter methanhaltigem Gas werden die in Erdöl- und Erdgasfeldern abgefackelten Gase mit einem Anteil von wenigstens 50 % CH₄ verstanden. Je nach dem Vorkommen der ölhaltigen Schichten wird die Methankonzentration in den zurzeit abgefackelten Gasen unterschiedlich sein und kann innerhalb von 30 bis 86 % schwanken (V.S. Arutyunov, O.V. Krylov Oxidative Umwandlungen von Methan. Institut für chemische Physik RAN. M. Nauka.1998.- 361 S).

Die Anlage zur homogenen Oxidation von methanhaltigem Gas ermöglicht es, den Nutzeffekt und die Wirtschaftlichkeit bei der Verwertung des genannten Gases zu steigern. Das wird durch einen gleichzeitigen Einsatz von Abhitzkesseln, Absorbern sowie einer Rohrleitung erreicht. Die Abhitzkessel sind für eine schnelle Abschreckung der Reaktionsmischung aus den Reaktoren für die homogene Oxidation des methanhaltigen Gases vorgesehen. Die Absorber sorgen für eine Reinigung des Umlaufgases und für eine Entfernung der sich ansammelnden Beimischungen und Nebenprodukte des Verarbeitungsprozesses, insbesondere SO₂, CO, CO₂. Die Rohrleitung ist eine zusätzliche Baugruppe. Sie verbindet die genannten Abhlitzkessel sowie einen der Wärmetauscher mit der Einrichtung zur Trennung (Rektifikation) der Produkte der homogenen Oxidation. Der genannte Wärmetauscher kühlt das Umlaufgas, welches die Oxidationsstufe des methanhaltigen Ausgangsgases verlässt.

Dieses bekannte Verfahren erreicht ähnliche Vorteile und verwendet die oben genannten Neuerungen. Nur wird das Umlaufgas vor seiner Förderung in den ersten Reaktor bis auf 430 - 450° C erwärmt. Danach erfolgen seine Kühlung, die Kondensation und die Ausscheidung der Reaktionsprodukte. Anschließend werden aus dem Umlaufgas SO₂, CO und CO₂ entfernt (Gasreinigung).

Ein bekanntes Verfahren zur Herstellung von Methanol umfasst
- eine getrennte Zuführung des bis auf 200 - 500° C vorgewärmten kohlenwasserstoffhaltigen Gases unter einem Druck von 2,5 - 15 MPa und eines sauerstoffhaltigen Gases in einen Mischraum,
- nachfolgende Produktionsstufen der unvollständigen Oxidation von Methan bei einer Sauerstoffkonzentration von 1 - 4 % (Vol.) mit zusätzlichem Einbringen von Reagenzien (Metalloxidkatalysator, höhere gasförmige Kohlenwasserstoffe oder sauerstoffhaltige Verbindungen, Kaltoxidationsmittel) in die Reaktionszone des Reaktors,
- eine Kühlung der Reaktionsmischung in einem Wärmetauscher,
- eine Ausscheidung von Methanol aus den flüssigen Reaktionsprodukten im Abscheider und
- eine Abführung der gasförmigen Reaktionsabprodukte in den Eintritt des Reaktors.
   (s. RU, 2049086 Cl , C 07 C 31/04, 27.11.1995).

Der Mangel dieses Verfahrens besteht darin, dass es notwendig ist, einen Katalysator oder zusätzliche Reagenzien zu benutzen und die Reaktionsgase sehr stark zu erhitzen. Das verursacht eine verminderte Methanolausbeute und eine erhöhte Wahrscheinlichkeit einer Rußbildung.

Aus der GB, 2196335 A, C 07 C 31/04, 27.04.1988 ist ein Verfahren zur Herstellung von Methanol bekannt. Dieses Verfahren umfasst
- eine getrennte Zuführung des kohlenwasserstoffhaltigen Gases (Erdgas oder Methan) und des sauerstoffhaltigen Gases (Luft oder Sauerstoff) in einen Mischer,
- eine nachfolgende Zuführung der Mischung in einen inerten Reaktor,
- eine unvollständige Gasphasenoxidation des kohlenwasserstoffhaltigen Gases in einem Reaktor unter einem Druck von 1 - 10 MPa im Laufe von 2 - 1000 Sek. bei einer Temperatur von 300 - 500° C ohne Katalysator, wobei der Sauerstoffgehalt 2 - 20 % (Vol.) beträgt,
- eine Ausscheidung von Methanol aus den Reaktionsprodukten in einem Verdichter und
- eine Rückführung der das nichtreagierte Methan enthaltenden Reaktionsabgase zur Vermengung mit dem kohlenwasserstoffhaltigen Grundgas in den ersten Reaktor bzw. in den zweiten Reaktor, welcher an den ersten Reaktor nachfolgend angeschlossen ist.

Der Mangel dieses Verfahrens ist eine große Reaktionsdauer, welche die Leistung des Reaktors bei der Methanolproduktion begrenzt.

Eine bekannte Anlage zur Herstellung von Methanol weist nacheinander aufgestellt und mittels Verrohrung verbunden einen Mischraum, einen Reaktor, einen Verdichter und einen Abscheider sowie einen Behälter auf. Der Mischraum ist an die getrennten Versorgungen von kohlenwasserstoffhaltigem Gas und Luft oder Sauerstoff angeschlossen. Der Reaktor ist aus einem inerten Material gefertigt und weist Heizelemente für eine unvollständige Oxidation von Methan in der Mischung auf, welche dem Reaktor unter Überdruck zugeführt wird. Der Verdichter und der Abscheider dienen zur Ausscheidung von Methanol aus den Reaktionsprodukten. Der Behälter ist für die gasförmigen Reaktionsumlaufprodukte vorgesehen. Der Behälter hat eine Rohrleitung, um die gasförmigen Reaktionsumlaufprodukte dem kohlenwasserstoffhaltigen Ausgangsgas oder dem Mischraum zuzuführen (GB, 2196335 A, C 07 C 31/04, 27.04,1988). Das Verfahren und die Anlage stellen eine hohe Ausbeute von Methanol sicher. Dabei können 5 bis 15 % des Methans bei jeder Passage eines Reaktors reagieren. Jedoch wird die hohe Leistung der Anlage durch eine lange Aufenthaltszeit der Reagenzien im Reaktor begrenzt.

Aus der SU 1469788 Al, C 07 C 31/04, 20.11.96 ist ein Verfahren zur Herstellung von Methanol mittels einer getrennten Förderung des kohlenwasserstoffhaltigen Gases und seiner Oxidation mittels eines sauerstoffhaltigen Gases bei 370 - 450° C und 5 - 20 MPa bekannt. Dabei beträgt die Kontaktzeit der Gase im Reaktor 0,2 - 0,22 Sek. Die erwärmte Reaktionsmischung wird dann bis auf 330 - 340° C abgekühlt, wobei Methanol dem Reaktor zugeführt wird. Oder es wird die Reaktionsmischung ohne Zwischenkondensation und Separation bis auf 380 - 400° C in den Zwischenstufen-Wärmetauschern abgekühlt, welche im Reaktor eingebaut sind. Danach kommt das Reaktionsgemisch in 2 bis 3 aufeinander folgende Oxidationsstufen (SU, 1336471 Al, C 07 C 31/04, 27.09.96).

Der Mangel dieses Verfahrens besteht in einem unvermeidlichen Mehrverbrauch von Methanol und seiner unumgänglichen erneuten Ausscheidung. Das hängt mit seinen Verlusten zusammen, die auch nicht vermieden werden können. Oder es müssen zusätzliche Kühlungskreisläufe eingerichtet werden, wobei das zusätzliche Kühlmedium umlaufen sollte.

Aus der RU 2057745, 10.04.1996 sind ein Verfahren zur Herstellung von Methanol und eine Anlage dafür bekannt. Das Verfahren umfasst eine getrennte Förderung des konsequent verdichteten und erwärmten kohlenwasserstoffhaltigen (Erd-) Gases und der Druckluft oder des Sauerstoffs in eine Mischzone (Mischeinheit) eines Reaktors; die folgerichtige stufenweise Oxidation des kohlenwasserstoffhaltigen Gases bei einer Anfangstemperatur von 325 - 500° C, einem Druck von 3 - 10 MPa und einem Sauerstoffgehalt von 1,5 - 8 % (Vol.) in zwei Reaktionszonen des Reaktors unter einer zusätzlichen Versorgung der nachfolgenden Mischzone mit Sauerstoff oder Luft, Kühlung der Reaktionsmischung, Ausscheidung von Methanol aus dem gekühlten Reaktions-Gas-Flüssigkeitsgemisch und Zuführung der Abgase in das kohlenwasserstoffhaltige Grundgas (Rohrleitung) oder eine Verbrennung dieser Abgase.

Diese Anlage zur Herstellung von Methanol weist auf: montierte und an die Versorgung des kohlenwasserstoffhaltigen Gases angeschlossene Verdichter, Rekuperativwärmetauscher und direkte Feuererhitzer für die Verflüssigung und die Erhitzung des kohlenwasserstoffhaltigen Gases, eine an den Verdichter angeschlossene getrennte Sauerstoff- oder Luftversorgung, einen Reaktor mit zwei Misch- und Reaktionszonen mit Rohrleitungen für die getrennte Förderung des kohlenwasserstoffhaltigen Gases und Luft oder Sauerstoff in die Mischzonen, die nacheinander aufgestellt und mit der Reaktionszone des Reaktors verbunden sind, einen rekuperativen Röhrenwärmetauscher zur Kühlung der Reaktionsmischung und zur Erhitzung des kalten kohlenwasserstoffhaltigen Gases, einen Kühler-Verdichter, einen Abscheider zur Ausscheidung von Methanol aus dem gekühlten Reaktions- Gas-Flüssigkeitsgemisch und eine Rohrleitung für den Transport der Abgase aus dem Abscheider in die Rohrleitung des kohlenwasserstoffhaltigen Gases oder in den Reaktor.

Dieses bekannte Verfahren und die Anlage stellen keine ausreichend hohe Methanreformierung pro Passage von Erdgas durch den Reaktor sicher. Der wirksame Gasumlauf setzt fast unvermeidlich den Einsatz von Sauerstoff als Oxidationsmittel voraus. Seine Anwendung erhöht aber die Selbstkosten des Produkts um 20 bis 30 %. Darüber hinaus verringern die hohe Anfangstemperatur der Reaktion und die nachfolgende Selbsterhitzung der Reaktionsmischung die Ausbeute des Zielprodukts und tragen somit zu einer starken Rußbildung bei. Die Rußbildung erschwert den Betrieb der Produktionsanlagen und verringert die Qualität des erzeugten Methanols.

Der nächstkommende Stand der Technik gegenüber der Erfindung sind ein Verfahren und eine Anlage, die in der RU 2162460 (veröffentlicht am 27.01.2001) beschrieben sind. Diese bekannte Anlage zur homogenen Oxidation von Erdgas weist eine Erdgasversorgung, Wärmetauscher, Reaktoren, einen Abscheider, Sammelbehälter, eine Sauerstoffversorgung für die Gasphasen-Oxidation von Gas und Mittel zur Kühlung der Reaktionsmischung auf. Diese Einheiten sind miteinander mittels einer Hauptrohrleitung verbunden. Gemäß diesem bekannten Verfahren zur homogenen Oxidation von Erdgas werden das verdichtete und erwärmte Erdgas und das sauerstoffhaltige Gas getrennt zugeführt. Danach erfolgt die Oxidation des Erdgases mittels Sauerstoffs in Reaktoren mit nachfolgender Kühlung/Abschreckung. Dabei ist das Verfahren **dadurch gekennzeichnet, dass** das Reaktionsgemisch vor jeder nachfolgenden Oxidationsstufe um 70 - 150° C abgekühlt und in der letzten Reaktionszone abgeschreckt wird. Dabei wird die Temperatur der Reaktionsmischung um wenigstens 200° C innerhalb von weniger als 0,1 Sek. ihrer Verweilzeit in der Reaktionszone abgesenkt. Das Verfahren erfolgt zyklisch, wobei Umlaufgaseinblasungen und Ringschlüsse vorgenommen werden.

Der Mangel dieser bekannten Anlage ist ein niedriger Nutzeffekt. Der Mangel des Verfahrens sind eine geringe Ausbeute des Endprodukts und eine Verschmutzung des Umlaufgases durch Schwefel-Oxidationsprodukte (SO₂).

In diesem Verfahren und der Anlage sind aufeinander folgend einige Reaktoren aufgestellt. Die Wärmetönung davon ist benutzt, um in Abhitzkesseln Dampf zu erzeugen. Das Reaktionsgas wird bis zu einer optimalen Temperatur für den Eintritt in den nächsten Reaktor abgekühlt. Dadurch ist ein höherer Nutzeffekt bei der Umwandlung des Gases zu Methanol, Formaldehyd und Äthanol erreicht. Dieser bekannten Anlage ist ein immer zunehmender Anteil von Schwefeloxiden im Umlaufgas (SO₂) eigen. Das vermindert auch wesentlich den Umwandlungsgrad des Reaktionsgases, weil die Schwefeloxide kein Inertgas sind und in diesem Fall als Anti-Katalysatoren wirken.

Es ist Aufgabe der Erfindung, den Nutzeffekt bei der Anwendung des Verfahrens zu steigern, die Ausbeute des hergestellten Produkts zu erhöhen und die Ansammlungen von SO₂ im Umlaufgas zu entfernen. Darüber hinaus erweitert die Erfindung die Auswahl an Verfahren und Anlagen zur homogenen Oxidation von methanhaltigem Gas.

Die gestellten Aufgaben werden durch die Merkmale der Ansprüche 1 und 2 gelöst.

Der Umwandlungsgrad bei der Umwandlung des Reaktionsgases in Produkte der homogenen Oxidation in einem einzigen Reaktor ist nicht hoch (min. 2 %, max. 4 %, je nach Gaszusammensetzung und nach Wahl der optimalen Temperatur und des optimalen Drucks). Deswegen kann eine wirtschaftlich bedingte Anzahl der Reaktoren mindestens drei betragen. Ihre maximale Menge wird durch die ursprüngliche Gaszusammensetzung festgelegt.

Darüber hinaus ist es verfahrenstechnisch sinnvoll, den Austritt des letzten Absorbers (nach der Entfernung von SO₂, CO₂ und CO aus dem Umlaufgas) mit der Hauptrohrleitung zu verbinden.

Das genannte technische Ergebnis wird in der neuen Anlage folgenderweise erreicht: Die Anlage schließt eine Versorgung des methanhaltigen Gases, Wärmetauscher, wenigstens drei Reaktoren, einen Abscheider und Rohrleitungen ein. Diese Einheiten sind untereinander mittels einer Hauptrohrleitung verbunden. Dabei sind die Reaktoren aus Kohlenstoffstahl gefertigt. Dabei ist jeder der Reaktoren, bis auf den letzten, jeweils unabhängig mit einem Abhitzkessel verbunden. Die Reaktoren sind auch mit Sauerstoffversorgungen verbunden. Auf der Strecke vom letzten mit dem Abhitzkessel nicht verbundenen Reaktor zum Abscheider gibt es Wärmetauscher, welche das Umlaufgas aufwärmen und die Erzeugung von Wasserdampf sicherstellen. Der Wasserdampf wird mit dem Dampf aus den Abhitzkesseln vereinigt, und zwar mittels einer zusätzlichen Rohrleitung, welche den Dampf in die Rektifikationssäule zur Trennung der Endprodukte fördert. Die flüssige Phase wird aus dem Abscheider über Rohrleitungen zur Rektifikationsstufe gefördert. Hier werden Methanol-Rektifikat, Äthylalkohol, Formaldehyd erzeugt. Die gasförmige Phase kommt zur Reinigung und zur Entfernung von SO₂, CO₂ und CO in die nacheinander folgend angeordneten Absorber. Hier sind die Austritte für die flüssige Phase der Absorber mit Sammelbehältern verbunden. Dies ermöglicht eine Rückgewinnung der Lösungen unter Ausscheidung und Anführung der CO- und CO₂-Fraktionen. Der Austritt des letzten Absorbers ist mit der Hauptrohrleitung bis zur Anordnungsstelle eines Umlaufgas-Hochdruckgebläses verbunden. Das stellt die Möglichkeit eines teilweisen Abblasens des Umlaufzyklus sicher, um evtl. vorhandene Inertstoffe abzuleiten.

Das technische Ergebnis gemäß dem neuen Verfahren wird folgenderweise erreicht: Das methanhaltige Gas wird bis auf 430 - 450° C vorgewärmt und in wenigstens drei in Reihe angeordnete Oxidationsreaktoren gefördert. Die Reaktoren sind aus Kohlenstoffstahl gefertigt. Dabei ist jeder der Reaktoren, bis auf den letzten, jeweils unabhängig mit einem Abhitzkessel verbunden. Den Reaktoren wird auch Sauerstoff in einer solchen Menge zugeführt, dass ein Gemisch mit explosionsungefährlichen Konzentrationen entsteht. Das verursacht eine homogene Oxidation des methanhaltigen Gases unter gleichzeitiger Temperaturerhöhung der Gasmischung bis auf 540 - 560° C. Danach erfolgt eine schnelle Abschreckung/Kühlung der Gasmischung in den Abhitzkesseln bis auf 440 - 450° C. Hier entsteht der Dampf, welcher in die Rektifikationssäule zur Trennung der Endprodukte gefördert wird. Danach kommt das Reaktionsgemisch aus dem letzten mit keinem Abhitzkessel verbundenen Reaktor in den Abscheider. Dabei wärmt das Reaktionsgemisch auf seinem Weg zum Abscheider das Umlaufgas auf, und ein Teil dieser Hitze wird zur Herstellung von Wasserdampf verwendet. Der Wasserdampf wird mit dem Dampf aus den Abhitzkesseln vereinigt. Die flüssige Phase kommt aus dem Abscheider in die Rektifikationsstufe. Hier werden Methanol-Rektifikat, Äthylalkohol, Formaldehyd erzeugt. Die Gasphase wird zur Entfernung von SO₂, CO und CO₂ benutzt. Dabei wird gleichzeitig mit der Gasreinigung das teilweise Abblasen (Spülung) des Umlaufzyklus vorgenommen, um die Inertstoffe, zum Beispiel Stickstoff, Argon, zu entfernen. Die Anzahl der Abblasabläufe wird je nach dem Gehalt an Inertstoffen in einem Zyklus bestimmt. Nach der Reinigung und dem Abblasen erfolgt der Rückschluss des Zyklus mittels Zuspeisung der Gasphase/des Umlaufgases in das methanhaltige Grundgas. Das neu erzeugte Gas wird auch wieder dem Reaktor zugeführt.

Ein Ausführungsbeispiel der Erfindung ist in der Zeichnung schematisch dargestellt. Es zeigt:

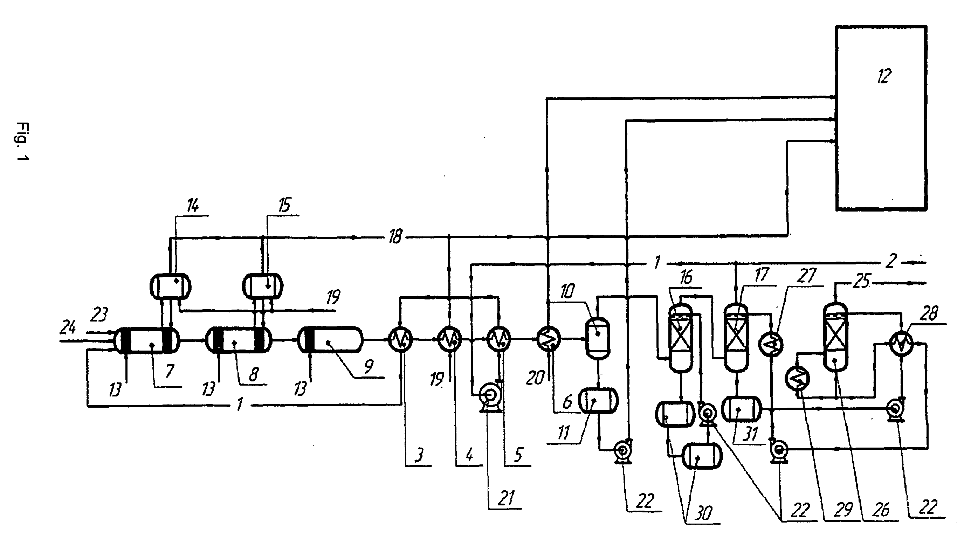

- Fig. 1: das Schaltbild der Anlage zur Durchführung des Verfahrens zur homo- gen Oxidation von methanhaltigem Gas.

Die Anlage zur homogenen Oxidation von methanhaltigem Gas nach Fig. 1 weist die miteinander mittels einer Hauptrohrleitung 1 verbundenen Einheiten auf:
- eine Gasversorgung 2 für die Zuführung von methanhaltigem Gas,
- Wärmetauscher 3, 4, 5 und 6,
- aufeinander folgend angeordnete Reaktoren 7, 8 und 9,
- einen Abscheider 10,
- Sammelbehälter 11, 30 und 31,
- eine Sauerstoffversorgung 13 für die nichtkatalytische Gasphasen- Oxidation von Gas, und
- Mittel zur Kühlung der Reaktionsmischung.

Die Anlage weist auch auf:
- Abhitzkessel 14 und 15,
- Absorber 16 und 17,
- eine zusätzliche Rohrleitung 18, die die Austritte der Abhitzkessel 14 und 15 und einen der Austritte des Wärmetauschers 4 verbindet, um den gebildeten Dampf in der Rektifikationsstufe während der Rektifikation der Reaktionsprodukte der homogenen Oxidation zu benutzen.

Dabei sind die Reaktoren 7, 8 und 9, die mit der Sauerstoffversorgung 13 verbunden sind, aus Kohlenstoffstahl gefertigt. Sie dienen der Vermeidung eines teilweisen Abbaus der Produkte der homogenen Oxidation.

Der Wärmetauscher 3 ist so ausgebildet, dass er die Aufwärmung des Umlaufgases ermöglicht. Sein Eintritt ist mit dem Austritt des Wärmetauschers 5 verbunden, dessen Eintritt mit einem Umlaufgasgebläse 21 verbunden ist. Dabei ist der Austritt des Wärmetauschers 3 mit dem Eintritt des Reaktors 7 verbunden. Dadurch ist die Möglichkeit der Bildung von primären Produkten der homogenen Oxidation des methanhaltigen Gases sichergestellt. Die Reaktoren 7, 8 und 9 sind der Reihe nach miteinander mittels der Hauptrohrleitung 1 verbunden. Das stellt die Möglichkeit der Bildung von sekundären Produkten der homogenen Gasoxidation sicher. Die mit Hilfe von Kühlwasser aus einer Kühlwasserleitung 20 beim Fließen durch den Wärmetauscher 6 abgeleitete Wärme ist benutzt, um die Oxidationsprodukte bei ihrer Förderung in die erste Rektifikationssäule (nicht abgebildet) der Reflektionseinheit 12 zur Trennung in ihre Bestandteile zu erwärmen.

Der Austritt des letzten Reaktors 9 wird mit dem zweiten Eintritt des Wärmetauschers 3 verbunden, um das hinein fließende Gas aus der methanhaltigen Gasversorgung 2 zusätzlich bis auf eine Temperatur von 430 - 450° C aufzuwärmen.

Die Wärmetauscher 3, 4, 5 und 6 werden nacheinander miteinander verbunden. Der letzte Wärmetauscher 6 ist so ausgebildet, dass die endgültige Kühlung und Kondensation der Oxidationsprodukte ermöglicht ist. Sein Eintritt ist mit der Kühlwasserleitung 20 verbunden, und sein Austritt ist mit dem Eintritt eines Abscheiders 10 verbunden. Einer der Austritte des Abscheiders 10 (flüssiges Kondensat) ist mit einem der Sammelbehälter 11 verbunden, um nachher daraus die verdichteten Produkte mittels einer Pumpe 22 in die Rektifikationsstufe zur Herstellung von Methanol-Rektifikat, Äthylalkohol, Formaldehyd umzupumpen. Der andere Austritt des Abscheiders 10 (nicht verdichtete Gase) ist mit einem der Eintritte des ersten Absorbers 16 verbunden. Das stellt die Möglichkeit der Entfernung von SO₂ aus dem Umlaufgas darin sicher. Nach dem Absorber 16 sind die Gase weiter zum Eintritt in den zweiten Absorber 17 gefördert. Hier ist die Entfernung von CO- und CO₂-Fraktionen aus dem Umlaufgas ermöglicht.

Die Austritte für die flüssige Phase der Absorber 16 und 17 sind jeweils mit Sammelbehältern 30 und 31 verbunden. Damit ist die Möglichkeit zur Rückgewinnung der Absorbierlösungen und die Ausscheidung und Ableitung der CO- und CO₂-Fraktionen aus einer Desorptionseinrichtung 26 sichergestellt. Die Austritte der Sammelbehälter 30 und 31 sind mit den Eintritten der Absorber 16 und 17 verbunden, um ihnen die gebildeten Lösungen zur Entfernung von SO₂, CO und CO₂ aus dem Umlaufgas (Umlaufgasreinigung) zuzuführen. Der Austritt des zweiten Absorbers 17 ist mit der Hauptrohrleitung 1 bis zur Stelle des Umlaufgasgebläses 21 verbunden, um die Möglichkeit eines teilweisen Abblasens des Umlaufzyklus sicherzustellen und evtl. vorhandene Inertstoffe abzuleiten.

Die Mittel zur Kühlung der Reaktionsmischung umfassen die Wärmetauscher 3, 4, 5 und 6 und die Abhitzkessel 14 und 15.

Eine Speisewasserversorgung 19 ist mit den Abhitzkesseln 14 und 15 verbunden. Die Speisewasserversorgung 19 ist auch mit dem Wärmetauscher 4 zwecks Dampfbildung verbunden.

Das Bezugszeichen 23 zeigt das Einströmen der Methanol-Fraktion, das Bezugszeichen 24 das Einströmen des methanhaltigen Gases, das Bezugszeichen 25 den Austritt der CO- und CO₂-Fraktionen für eine weitere Anwendung. Die Behälter 30 und 31 sind für die Absorptionslösungen vorgesehen. Die Pumpen (22), die Wärmetauscher/Kühler (27), der rekuperative Wärmetauscher (28) und die Wärmetauscher/Erhitzer (29) sind für den Rückschluss des neuen Gases verwendet.

Beschreibung der Funktionsweise der Anlage zur homogenen Oxidation von methanhaltigem Gas und des Verfahrens zur homogenen Oxidation:
Der Vorgang der homogenen Oxidation von methanhaltigem Gas mit der Erzeugung von Methanol ist aufgrund der Bedingungen zur Senkung des Energieaufwands im Zusammenhang mit einer Verdichtung des methanhaltigen Gases unter Kreislaufführung in folgender Weise durchgeführt: Das im Wärmetauscher 3 bis auf eine Temperatur von 450° C aufgewärmte Umlaufgas kommt in den Reaktor 7. Hier wird es mit Sauerstoff im Verhältnis unter der explosionsgefährlichen Grenze vermengt. Der Sauerstoff oxidiert teilweise das Methan und andere Kohlenwasserstoffe, die im Grundgas enthalten sind. Dabei steigt die Temperatur der Gasmischung bis auf 540 - 560° C an. Es werden Methanol, Äthanol, Formaldehyd gebildet. Die Reaktion der Bildung von Produkten der homogenen Oxidation ist umkehrbar. Folglich muss eine schnelle Abschreckung/ Kühlung der Reaktionsmischung von 540 - 560° C auf 440 - 450° C vorgenommen werden. Dies erfolgt in den Abhitzkesseln 14 und 15 und dem Wärmetauscher 3.

Die Anwesenheit von Nickel und seiner Verbindungen (nichtrostende Stähle) in der Reaktionszone verursacht im Allgemeinen einen teilweisen Abbau der Produkte der homogenen Oxidation. Um dieses Phänomen zu vermeiden, sind die Reaktoren 7, 8 und 9 aus Kohlenstoffstahl gefertigt.

Nach dem Reaktor 7 kommt das Umlaufgas in den Reaktor 8. Hier wiederholt sich der Vorgang der homogenen Oxidation von Methan ähnlich wie im Reaktor 7. Danach erfolgt ein ähnlicher Vorgang im Reaktor 9. Danach wärmt das Umlaufgas im Wärmetauscher 3 das zum Reaktor 7 fließende Gas auf.

Das gebliebene Wärmepotential des Umlaufgases ist im Wärmetauscher 4 zwecks Dampferzeugung abgenommen. Dieser Dampf ist zusammen mit dem Dampf aus den Abhitzkesseln 14 und 15 während der Rektifikationsstufe für Produkte der homogenen Oxidation verwendet.

Nach dem Wärmetauscher 4 wärmt das Umlaufgas im Wärmetauscher 5 das über den Wärmetauscher 3 zum Reaktor 7 fließende Gas auf. Die endgültige Kühlung und Kondensation der Oxidationsprodukte ist im Wärmetauscher 6 mit Hilfe von Kühlwasser vorgenommen.

Die verdichteten Produkte der homogenen Oxidation werden im Abscheider 10 getrennt und in den Sammelbehälter 11 abgeleitet. Von da aus kommen sie in die Rektifikationsstufe. Hier werden sie getrennt, wobei Methanol-Rektifikat, Äthylalkohol und Formaldehyd hergestellt werden.

Das Umlaufgas fließt aus dem Abscheider 10 in die Absorber 16 und 17. Hier wird das Umlaufgas gewaschen (gereinigt). Das heißt, im ersten Absorber 16 wird SO₂ und im zweiten Absorber 17 werden CO und CO₂ entfernt. Die Absorptionslösung des zweiten Absorbers 17 wird in der Desorptionseinrichtung 26 aufgefrischt, wobei daraus CO und CO₂ ausgeschieden werden. Die im Wärmetauscher 27 abgekühlte Lösung wird wieder zur Umlaufgasreinigung zurückgeführt.

Die Ausscheidung von CO und CO₂ aus der Gaskreislaufführung ist aufgrund der Bedingung zur Verringerung des Sauerstoffverbrauchs um 15 % sowie zur Abnahme von Anteilen von inerten SO₂ und CO₂ im Zyklus erforderlich. Der Sauerstoff wird für die Nachoxidation von CO zu CO₂ benötigt.

Anschließend ist das teilweise Abblasen des Umlaufzyklus vorgesehen, um die in den Zyklus zusammen mit dem methanhaltigen Grundgas und Sauerstoff eingetretenen inerten Gase (Stickstoff, Argon, Krypton usw.) abzuführen. Die Größe des Abblasens wird je nach Bilanz aufgrund der zulässigen Menge von Inertstoffen im Zyklus bestimmt. Die Menge der Inertstoffe kann im Umlaufgas 70 % erreichen. Eine weitere Zunahme des Gehalts an Inertstoffen verursacht eine Verminderung des Umwandlungsgrads des Reaktionsgases.

Nach dem Abblasen des Zyklus wird das Reaktionsgas in das methanhaltige Gas eingespeist. Danach wird das Umlaufgas mittels des Umlaufgasgebläses 21 in die Wärmetauscher 5, 4 und 3 sowie zum Eintritt in den Reaktors 7 gefördert. Damit ist der Arbeitszyklus der Anlage vollständig beschrieben.

**Tabelle 1**

| | Umwandlungsgrad des Reaktionsgases, % |
|---|---|
| Bekanntes Verfahren und Anlage | 6,0 |
| Neues Verfahren und Anlage | 9,0 |

Die Angaben über den Umwandlungsgrad innerhalb eines Zyklus im Funktionsschema mit drei nacheinander aufgestellten Reaktoren gemäß den in einem einzigen Reaktor erfassten Versuchsdaten sind der Tabelle 1 zu entnehmen.

Die Erfindung erhöht den Nutzeffekt während des Betriebs und steigert die Ausbeute des hergestellten Produkts.

Die Erfindung kann in der petrolchemischen Industrie, zur Verwertung von abgefackelten Gasen bei der Herstellung von Methanol, Formaldehyd und anderen Produkten eingesetzt werden.

### Bedeutung der Bezugszeichen im Arbeitsschema:

- 1 -: Hauptrohrleitung;
- 2 -: Gasversorgung;
- 3, 4, 5, 6 -: Wärmetauscher;
- 7, 8, 9 -: Reaktoren für eine homogene Oxidation;
- 10 -: Abscheider für die Oxidationsprodukte (Oxidat);
- 11 -: Sammelbehälter für die Oxidationsprodukte (Oxidat);
- 12 -: Rektifikationseinheit für die Oxidationsprodukte;
- 13 -: Sauerstoffversorgung;
- 14, 15 -: Abhitzkessel;
- 16, 17 -: Absorber mit Wirbelpaketeinbauten;
- 18 -: Rohrleitung für rekuperative Wärme (Dampf);
- 19 -: Speisewasserversorgung;
- 20 -: Kühlwasserleitung;
- 21 -: Umlaufgasgebläse;
- 22 -: Pumpen für die Oxidationsprodukte (Oxidat) der Absorber und Desorp- tionseinrichtungen;
- 23 -: Einströmen der Methanolfraktion;
- 24 -: Startgas;
- 25 -: CO- und CO₂-Fraktionen;
- 26 -: Desorptionseinrichtung;
- 27 -: Wärmetauscher - Kühler des Absorptionsmittels;
- 28 -: rekuperativer Wärmetauscher;
- 29 -: Wärmetauscher - Rektifikator;
- 30,31 -: Sammelbehälter für Absorptionslösungen

## Patentansprüche

1. Verfahren zur homogenen Oxidation von methanhaltigem Gas, wobei das verdichtete und erwärmte Gas und sauerstoffhaltiges Gas getrennt zugeführt, die Oxidation in Reaktoren mit nachfolgender Kühlung durchgeführt, das Reaktionsgemisch abgekühlt und in der letzten Oxidationsstufe abgeschreckt werden, **dadurch gekennzeichnet,**
**dass** die Förderung des bis auf 430 - 450° C vorgewärmten methanhaltigen Gases über wenigstens drei in Reihe angeordnete Oxidationsreaktoren (7, 8, 9) aus Kohlenstoffstahl vorgenommen wird, wobei diese Reaktoren (7, 8), bis auf den letzten (9), mit Abhitzkesseln (14, 15) verbunden sind,
**dass** den Reaktoren (7, 8) Sauerstoff in einer solchen Menge zugeführt wird, um ein Gemisch mit einer explosionsmäßig ungefährlichen Konzentration zu bilden, was die homogene Oxidation von methanhaltigem Gas unter gleichzeitiger Temperaturerhöhung der Gasmischung bis auf 540 - 560° C hervorruft,
**dass** danach eine schnelle Abschreckung/Kühlung der Gasmischung in den Abhitzkesseln (14, 15) bis auf 440 - 450° C vorgenommen wird, wobei Dampf gebildet wird, der in die Rektifikationssäule (12) zur Trennung der Endprodukte gefördert wird,
**dass** das Reaktionsgemisch aus dem letzten Reaktor (9), der mit keinem Abhitzkessel verbundenen ist, in einen Abscheider (10) geleitet wird,
**dass** das Reaktionsgemisch auf dem Weg zum Abscheider (10) das Umlaufgas aufwärmt, wobei ein Teil dieser Hitze zur Herstellung von Wasserdampf verwendet wird,
**dass** der Wasserdampf mit dem Dampf aus den Abhitzkesseln (7, 8) vereinigt wird,
**dass** die flüssige Phase aus dem Abscheider (10) in die Rektifikationsstufe (12) geleitet wird, wobei Methanol-Rektifikat, Äthylalkohol, Formaldehyd erzeugt und die Gasphase zur Reinigung und Entfernung von SO₂, CO und CO₂ weitergeleitet werden,
**dass** dabei gleichzeitig mit der Gasreinigung ein teilweises Abblasen des Umlaufzyklus vorgenommen wird, um die Inertstoffe, z. B. Stickstoff, Argon, zu entfernen,
**dass** die Anzahl der Abblasabläufe je nach zulässigem Gehalt an Inertstoffen in einem Zyklus bestimmt wird,
**dass** nach der Reinigung und dem Abblasen der Rückschluss des Zyklus mittels der Zuspeisung der Gasphase/des Umlaufgases mit dem methanhaltigen Gemisch vorgenommen wird und
**dass** das neu erzeugte Gas wieder dem Reaktor (7) zugeführt wird.

2. Anlage zur homogenen Oxidation von methanhaltigem Gas zur Durchführung des Verfahrens nach Anspruch 1,
**dadurch gekennzeichnet,**
**dass** hintereinander geschaltete Wärmetauscher (3, 4, 5) über eine Hauptrohrleitung (1) mit methanhaltigem Gas versorgt sind,
**dass** wenigstens drei Reaktoren (7, 8, 9) aus Kohlenstoffstahl verwendet sind, wobei die Reaktoren (7, 8), bis auf den letzten Reaktor (9), mit Abhitzkesseln (14, 15) verbunden sind,
**dass** die Reaktoren (7, 8, 9) mit Sauerstoffversorgungen (13) verbunden sind, dass die Anlage einen Abscheider (10) aufweist, wobei zwischen dem letzten Reaktor (9) und dem Abscheider (10) ein Wärmetauscher (6) vorgesehen ist, der das Umlaufgas aufwärmt und die Erzeugung von Wasserdampf sicherstellt, dass der Wasserdampf mit dem Dampf aus den Abhitzkesseln (14, 15) vereinigt ist und zwar über eine zusätzliche Rohrleitung, die den Dampf der Rektifikationssäule (12) zur Trennung der Endprodukte zuführt,
**dass** die flüssige Phase über Rohrleitungen aus dem Abscheider (10) zur Rektifikationsstufe (12) förderbar ist, wobei Methanol-Rektifikat, Äthylalkohol, Formaldehyd erzeugbar sind,
**dass** die gasförmige Phase zur Entfernung von SO₂, CO und CO₂ in hintereinander geschaltete Absorber (16, 17) förderbar ist, wobei die Austritte der Absorber (16, 17) für die flüssige Phase mit Sammelbehältern (30, 31) verbunden sind, um die Rückgewinnung der Lösungen unter Ausscheidung und Abführung von CO- und CO₂-Fraktionen sicherzustellen und
**dass** der Austritt des letzten Absorbers (17) mit der Hauptrohrleitung (1) bis zur Einbaustelle eines Umlaufgas-Gebläses (21) verbunden ist, um ein teilweises Abblasen des Umlaufzyklus sicherzustellen und evtl. vorhandene Inertstoffe abzuleiten.
